# EUROPEAN PATENT APPLICATION

(11) **EP 4 599 820 A1**
(43) Date of publication of application: **13.08.2025**
(21) Application number: 23876523.4
(22) Date of filing: 26.09.2023
(51) Int. Cl.: A61K 9/00, A61M 37/00, A61K 47/36, A61K 47/38, A61K 47/58

(54) **MICRONEEDLE FORMULATION AND PREPARATION METHOD FOR MICRONEEDLE PATCH**

(30) Priority: 09.10.2022 CN 202211227666
(71) Applicant: Shenzhen Qinglan Biotechnology Co., Ltd., Shenzhen, Guangdong 518000 (CN)
(72) Inventor: JIANG, Lin, Shenzhen, Guangdong 518000 (CN)
(74) Representative: Herrero & Asociados, S.L.
(86) International application number: PCT/CN2023/121298
(87) International publication number: WO 2024/078319

(57) **Abstract**

A microneedle formulation is provided, which comprises polyvinyl alcohol, polyvinylpyrrolidone and hydroxyethyl cellulose. The polyvinyl alcohol comprises a first polyvinyl alcohol and a second polyvinyl alcohol, wherein the viscosity of the first polyvinyl alcohol is 2.4-12.3 mPas, and the viscosity of the second polyvinyl alcohol is 17.2-42.6 mPas; The polyvinylpyrrolidone comprises a first polyvinylpyrrolidone and a second polyvinylpyrrolidone, wherein the molecular weight of the first polyvinylpyrrolidone is 3500-150000, and the molecular weight of the second polyvinylpyrrolidone is 210000-1500000. The microneedle formulation can effectively improve the structural strength of microneedles. Meanwhile, the invention also provides a preparation method of the microneedle patch.

## Description

### Technical field

The present application belongs to the technical field of pharmaceutical preparations, and particularly relates to a microneedle formulation and a preparation method for a microneedle patch.

### Background

Soluble microneedles have gained popularity in the microneedle industry due to their inexpensive production costs, high biological stability, convenience of use, and effective management of the drug release curve. Unlike solid and hollow needles, soluble microneedles can remain in the skin with only one shoot while controlling the drug release rate, which is mostly determined by the microneedle's structure. Therefore, modifying the composition of the microneedle formulation allows for easy adjustment of the drug release rate.

However, soluble microneedles do have several flaws. For example, soluble microneedles use water-soluble molecules as an excipient, and their mechanical strength and physical stability in storage differ significantly from other forms of microneedles. The existing soluble microneedle formulation has insufficient mechanical strength, which results in insufficient penetration depth when applied to the skin, and it is easy to break when not penetrated to the target depth, which further affects the dosage and is not conducive to maintaining the stability of the dosage. Furthermore, the current production efficiency of soluble microneedles, particularly the dehydration efficiency, is low, and the API contained in soluble microneedles is prone to losing its physical, chemical, and biological activities due to excessively high temperatures and long dehydration duration. As a result, the mechanical strength of soluble microneedles is strongly impacted by the degree of dehydration, and whether the dehydration circumstances are harsh or moderate has a significant impact on the product quality of soluble microneedles.

Therefore, finding an appropriate soluble microneedle formulation and a gentle yet effective dehydration procedure is crucial for increasing production efficiency and quality control of soluble microneedles.

### Summary of the invention

In order to solve the problems of the current soluble microneedles' low production efficiency and insufficient mechanical strength, which affect the quality of administration, the present application provides a microneedle preparation and a preparation method for microneedle patch.

The technical solutions adopted by the application to solve the technical problems are as follows.

In one aspect, the application provides a microneedle formulation, which includes a component A as follows:
polyvinyl alcohol, polyvinylpyrrolidone and hydroxyethyl cellulose;
wherein the polyvinyl alcohol includes a first polyvinyl alcohol and a second polyvinyl alcohol, and the first polyvinyl alcohol has a viscosity of 2.4-12.3 mPas, and the second polyvinyl alcohol has a viscosity of 17.2-42.6 mPas; and
wherein the polyvinylpyrrolidone includes a first polyvinylpyrrolidone and a second polyvinylpyrrolidone, the first polyvinylpyrrolidone has a molecular weight of 3500-150000, and the second polyvinylpyrrolidone has a molecular weight of 210000-1500000.

Optionally, the first polyvinylpyrrolidone has a viscosity of 0.8-10.6 mPas, and the second polyvinylpyrrolidone has a viscosity of 16.5-88.4 mPas.

Optionally, the component A includes the following components by weight:
2-6 parts of polyvinyl alcohol, 9.5-15 parts of polyvinylpyrrolidone and 5-7 parts of hydroxyethyl cellulose;
wherein, 0.5-2.5 parts of first polyvinyl alcohol and 1.5-3.5 parts of second polyvinyl alcohol; and
2.5-5 parts of first polyvinylpyrrolidone and 7-10 parts of second polyvinylpyrrolidone.

Optionally, the component A further includes 35-49 parts by weight of a first solvent.

Optionally, the microneedle formulation further includes a component B as follows:
sugar, buffer and salt.

Optionally, the component B includes the following components by weight:
11-16 parts of sugar, 0.3-0.8 parts of buffer and 0.5-2 parts of salt.

Optionally, the sugar includes 1-3 parts by weight of sucrose and 10-13 parts by weight of trehalose, and the component B further includes 0.002-0.01 part by weight of sodium hydroxide and 14-17 parts by weight of a second solvent.

Optionally, a weight ratio of the component A to the component B is (16.5-77): (11.8-35.81).

Optionally, the microneedle formulation further includes a pharmaceutical active ingredient, and a weight ratio of the component A to the pharmaceutical active ingredient is (16.5-77): (0.6-22.46).

In another aspect, the application provides a preparation method for microneedle patch, including the following steps:
obtaining a pharmaceutical active ingredient and the microneedle formulation described above, and preparing a molding liquid for microneedle molding;
applying the molding liquid on a microneedle molding die, dehydrating at -40-35°C until a water content is 3-15%, and demoulding to obtain a microneedle patch;
placing the microneedle patch in a protector and enclosing it in a sealed bag with a dehydrating agent to lower the water content of the microneedle patch to 3%. Preferably, the water content of the microneedle patch is less than 1%.

Optionally, the protector includes a bubble cap and a patch plate, wherein the bubble cap is arranged on the patch plate, and the microneedle patch is arranged in the bubble cap.

Optionally, the sealed bag has a water vapor permeability of less than 0.8 g/m²•24h.

Optionally, the sealed bag is a mixed film of one or more of biaxially oriented polypropylene, polyethylene terephthalate, chlorinated polypropylene resin and aluminum foil.

Optionally, the material of the bubble cap and/or the patch plate is a barrier material with a water vapor permeability of 1-20 g/m²•24h.

Optionally, the material of the bubble cap is polyvinyl chloride or polyethylene terephthalate.

Optionally, the material of the patch plate is a spunbonded olefin or an aluminum-plastic film.

In accordance with the microneedle formulation provided by the application, the inventor(s) conducted numerous experiments and discovered that adding hydroxyethyl cellulose with a specific molecular weight, first polyvinyl alcohol and second polyvinyl alcohol with a specific viscosity, and first polyvinylpyrrolidone and second polyvinylpyrrolidone with a specific molecular weight as skeleton materials -which serve as the formulation's primary strength-supporting components-can effectively increase the structural strength of the microneedles made using the invention; this will prevent the microneedles from breaking under extreme pressure and allow them to penetrate deeply into the skin to enhance the quality of administration. The cooperation between the first polyvinyl alcohol and the second polyvinyl alcohol within the above-mentioned viscosity range and the first polyvinylpyrrolidone and the second polyvinylpyrrolidone within the above-mentioned molecular weight range, is speculated to be due to the intertwining of polyvinyl alcohol and polyvinylpyrrolidone, and the length difference of their molecular chains plays a complementary role. This means that the first polyvinyl alcohol, second polyvinyl alcohol, first polyvinylpyrrolidone and second polyvinylpyrrolidone intertwine with one another to form a more stable supporting skeleton, so the overall strength of the microneedle is improved.

### Brief description of drawings

Fig. 1 is the result of a parafilm puncture experiment performed on a microneedle patch provided by embodiment 1 of the present application.
Fig. 2 is the result of a parafilm puncture experiment performed on microneedle patches provided by Comparative examples 1-4 to 4 of the present application.
Fig. 3 is the result of a leaf puncture experiment performed on a microneedle patch provided by embodiment 1 of the present application.
Fig. 4 is the result of a leaf puncture experiment performed on microneedle patches provided by Comparative examples 1-4 of the present application.
Fig. 5 is a morphological view of a microneedle patch provided by embodiment 1 of the present application before puncture.
Fig. 6 is a morphological view of a microneedle patch provided by embodiment 1 of the present application after puncture.
Fig. 7 is a morphological view of microneedle patches provided by Comparative examples 1-4 of the present application before puncture.
Fig. 8 is a morphological view of microneedle patches provided by Comparative examples 1-4 of the present application after puncture.
Fig. 9 is a schematic diagram of an integrated microneedle structure.
Fig. 10 is a structural schematic diagram of a layered microneedle.

### Detailed description of preferred embodiments

In order to make the technical problems, technical solutions and beneficial effects of the present application more clear, the application will be further explained in detail below with embodiments. It should be understood that the specific embodiments described here are only used to illustrate the application, rather than to limit the application.

In the description of the present application, the term "molecular weight" refers to the weight-average molecular weight.

The "barrier" in the term "barrier material" refers to the volume or weight of a plastic product with a certain thickness that permeates small molecular substances in a unit time (24h) and a unit area (1 m²) under a certain pressure (1 mPa) and a certain temperature (23°C), with a certain humidity.

An embodiment of the application provides a microneedle formulation, which includes a component A as follows:
polyvinyl alcohol, polyvinylpyrrolidone and hydroxyethyl cellulose;
wherein the polyvinyl alcohol includes a first polyvinyl alcohol and a second polyvinyl alcohol, and the first polyvinyl alcohol has a viscosity of 2.4-12.3 mPas, and the second polyvinyl alcohol has a viscosity of 17.2-42.6 mPas; and
wherein the polyvinylpyrrolidone includes a first polyvinylpyrrolidone and a second polyvinylpyrrolidone, the first polyvinylpyrrolidone has a molecular weight of 3500-150000, and the second polyvinylpyrrolidone has a molecular weight of 210000-1500000.

The inventor(s) conducted numerous experiments and discovered that adding hydroxyethyl cellulose with a specific molecular weight, first polyvinyl alcohol and second polyvinyl alcohol with a specific viscosity, and first polyvinylpyrrolidone and second polyvinylpyrrolidone with a specific molecular weight as skeleton materials -which serve as the formulation's primary strength-supporting components-can effectively increase the structural strength of the microneedles made using the invention; this will prevent the microneedles from breaking under extreme pressure and allow them to penetrate deeply into the skin to enhance the quality of administration. The cooperation between the first polyvinyl alcohol and the second polyvinyl alcohol within the above-mentioned viscosity range and the first polyvinylpyrrolidone and the second polyvinylpyrrolidone within the above-mentioned molecular weight range, is speculated to be due to the intertwining of polyvinyl alcohol and polyvinylpyrrolidone, and the length difference of their molecular chains plays a complementary role. This means that the first polyvinyl alcohol, second polyvinyl alcohol, first polyvinylpyrrolidone and second polyvinylpyrrolidone intertwine with one another to form a more stable supporting skeleton, so the overall strength of the microneedle is improved.

In the preferred embodiment, the viscosity of the first polyvinylpyrrolidone is 0.8-10.6 mPas, and the viscosity of the second polyvinylpyrrolidone is 16.5-88.4 mPas.

In some embodiments, the component A includes the following components by weight:
2-6 parts of polyvinyl alcohol, 9.5-15 parts of polyvinylpyrrolidone and 5-7 parts of hydroxyethyl cellulose;
wherein, 0.5-2.5 parts of first polyvinyl alcohol and 1.5-3.5 parts of second polyvinyl alcohol; and
2.5-5 parts of first polyvinylpyrrolidone and 7-10 parts of second polyvinylpyrrolidone.

The overall strength of the microneedles produced by the microneedle formulation can be preserved by restricting the addition amounts of the first polyvinyl alcohol and second polyvinyl alcohol with different viscosity and the first polyvinylpyrrolidone and second polyvinylpyrrolidone with different molecular weights. And when the addition amounts of the first polyvinyl alcohol, the second polyvinyl alcohol, the first polyvinylpyrrolidone and the second polyvinylpyrrolidone are not within the above range, the structural strength of the resulting microneedles will be deteriorated.

In some embodiments, the component A further includes 35-49 parts by weight of a first solvent.

The first solvent is used for dispersing the first polyvinyl alcohol, the second polyvinyl alcohol, the first polyvinylpyrrolidone, the second polyvinylpyrrolidone and the like in the component A.

Since the first polyvinyl alcohol, the second polyvinyl alcohol, the first polyvinylpyrrolidone and the second polyvinylpyrrolidone are all water-soluble molecules, in the preferred embodiment, the first solvent is selected from water, and more preferably, the first solvent is selected from water for injection.

Because the component A contains more high molecular weight substances, it is necessary to dissolve and disperse the component A under heating conditions, preferably at 65°C-95°C. In order to avoid denaturation of some temperature-sensitive substances caused by heating the component A, in some embodiments, the soluble microneedles also include component B, which can be added to the component B, and then mixed after the component A is cooled.

It should be noted that in other embodiment, if there is no temperature-sensitive substance in the microneedle formulation or component A is not heated, component A and component B mentioned in the present application can be mixed as the same component without special distinction.

In some embodiments, the component B includes sugars, buffers and salts.

In some embodiments, the component B includes the following components by weight:
11-16 parts of sugar, 0.3-0.8 parts of buffer and 0.5-2 parts of salt.

The sugar can be sucrose and/or trehalose, which is mainly used as a stabilizer in microneedle patches. It is a carbohydrate with good biocompatibility, high stability, low cost and good safety. The prepared microneedles have good mechanical strength, and can quickly release effective components in the skin when administered. When sucrose is absorbed by the skin, it is biodegradable in the body and will be gradually removed by the kidney.

The salt is selected from sodium chloride.

In some embodiments, the buffer is selected from sodium citrate, which is mainly combined with sodium chloride to form a buffer in the microneedle patch to prevent the PH value from fluctuating greatly in the preparation process.

In some embodiments, the sugar includes 1-3 parts by weight of sucrose and 10-13 parts by weight of trehalose. The component B further includes 0.002-0.01 part by weight of sodium hydroxide and 14-17 parts by weight of a second solvent.

Specifically, sucrose may also be replaced by other monosugars or disugars, such as maltose, lactose, fructose, etc.

The sodium hydroxide is used for adjusting the pH value of the microneedle formulation.

It should be noted that in the process of forming microneedles, it is necessary to remove the solvent by dehydration, and the first solvent and the second solvent are mostly removed at this time. Meanwhile, it is allowed to leave a small amount of solvent after dehydration, but excessive solvent residue will affect the strength of microneedles.

In some embodiments, the weight ratio of the component A to the component B is (16.5-77): (11.8-35.81).

In some embodiments, the microneedle formulation further includes a pharmaceutical active ingredient, and the weight ratio of the component A to the pharmaceutical active ingredient is (16.5-77): (0.6-22.46).

In some embodiments, the active ingredient of the drug is a small molecule drug or a biological drug, wherein the biological drug includes nucleic acid drugs such as DNA and RNA, protein, polypeptide and the like.

The pharmaceutically active ingredient can be selected from natural, synthetic or recombinant protein, peptides and fragments thereof. Representative examples of API types for delivery include antibiotics, antiviral drugs, painkillers, anesthetics, antihistamines, anti-inflammatory drugs, anticoagulants, allergens, vitamins, and antitumor drugs. Bioactive ingredients may include various hormones, vaccines for infectious diseases, therapeutic vaccines for cancer, nervous system diseases, allergies, smoking cessation or other addictions. For example, influenza antigen (influenza vaccine), HBs antigen (hepatitis B virus surface antigen), HBe antigen (hepatitis B Be antigen), BCG(Bacille de Calmette et Guerin) antigen, measles antigen, rubella antigen, varicella antigen, yellow fever antigen, herpes zoster antigen, rotavirus antigen, Hib (influenza B) antigen, rabies antigen, cholera antigen, diphtheria antigen, pertussis antigen, tetanus antigen, inactivated polio antigen, Japanese encephalitis antigen, human papillomavirus antigen or mixed antigen of the above viruses, etc..

In some embodiments, the microneedle formulation is used to prepare integrated microneedles or layered microneedles.

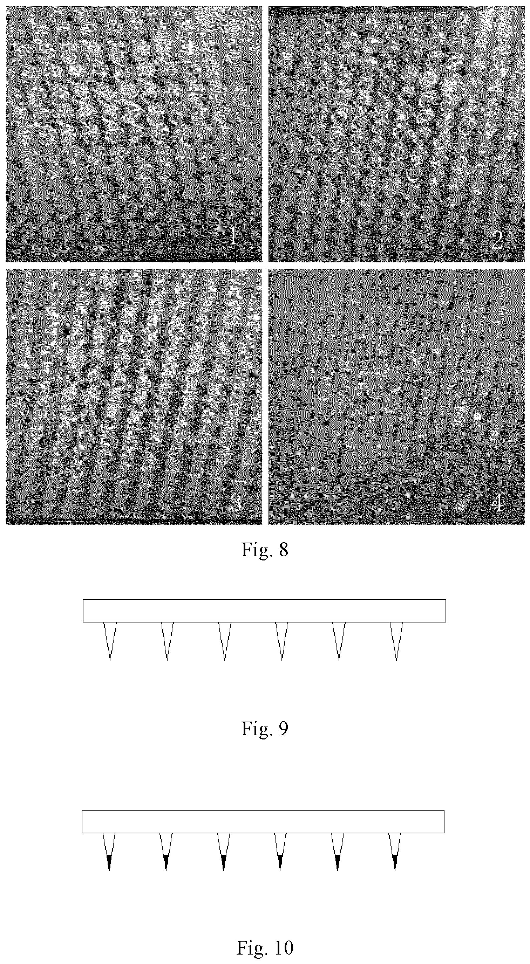

As shown in Fig. 9, when the prepared microneedles are integrated microneedles, the component A, component B and pharmaceutical active ingredient are directly mixed to obtain a microneedle formulation for preparing microneedles.

As shown in Fig. 10, when the prepared microneedles are layered microneedles, the component A and component B can be mixed to obtain a basement membrane liquid, and the medicinal active components are further added on the basis of part of the basement membrane liquid to obtain a needle tip liquid, which is used to form the needle tip part of the microneedle, and the basement membrane liquid is used to form the needle body part and basement membrane part of the microneedle.

Another embodiment of the present application provides a preparation method of the microneedle formulation as described above, which includes the following operation steps:
adding hydroxyethyl cellulose, the first polyvinyl alcohol, second polyvinyl alcohol, first polyvinylpyrrolidone and second polyvinylpyrrolidone into a first solvent, and dispersing by heating to obtain a component A.

In some embodiments, the heating temperature for dispersion is 65°C-95°C.

In some implementations, the following operations are also included:
dissolving sugar, buffer, sodium hydroxide and salt in a second solvent to obtain a component B; and
mixing the component A and component B to obtain a microneedle formulation.

In some embodiments, a pharmaceutical active ingredient is also added into the microneedle formulation.

In the dehydration stage of the preparation process of soluble microneedles, in order to prevent microneedles from being melted by heat or the materials such as active ingredients of drugs from being ineffective by heat, air drying dehydration is usually performed at a temperature below normal temperature. And meanwhile, too fast wind speed shall not be used in order to avoid microneedles from being deformed by wind. However, the dehydration rate is too slow under such dehydration conditions, especially when the water content of microneedles is at a low level, and the dehydration rate is slower than that in the previous period, making it insufficient for large-scale production. In addition, the low water content of the prepared microneedles is not conducive to the improvement of its structural strength.

In order to solve the above problems, another embodiment of the present application provides a preparation method for a microneedle patch, which includes the following steps:
obtaining a pharmaceutical active ingredient and the microneedle formulation of any one of claims 1 to 8, and preparing a molding liquid for microneedle molding;
applying the molding liquid on a microneedle molding die, dehydrating at -40-35°C until a water content is 3-15%, and demoulding to obtain a microneedle patch;
placing the microneedle patch in a protector and enclosing it in a sealed bag with a dehydrating agent to lower the water content of the microneedle patch to 3%.

The water vapor permeability of the sealed bag is less than 0.8 g/m²•24h, and it can be one or more mixed films of biaxially oriented polypropylene, polyethylene terephthalate, chlorinated polypropylene resin and aluminum foil.

By dehydrating the microneedle patch at -40-35°C in advance until the water content is 3-15%, the time for preliminary dehydration can be effectively reduced, the preparation speed can be accelerated, and the production cost can be reduced. If the time margin required for further dehydration is increasing, the humidity of 3-15% is taken as the critical point of preliminary dehydration. More importantly, under this water content condition, the obtained microneedle patch is not completely cured and has certain toughness. At this state, it is beneficial to demoulding the microneedle patch, avoiding the microneedle patch from being broken in the microneedle forming mold due to its own high rigidity during demoulding, and improving the morphological integrity of microneedles.

After preliminary dehydration, the microneedle patch is put into a protector and placed in a sealed bag with a dehydrating agent, which absorbs the moisture of the microneedle patch through the protector to reduce the moisture content of the microneedle patch to 3%. The dehydrating agent is a water-absorbent material such as molecular sieve or silica gel, and the protector can be used as a packaging piece of the microneedle patch, and the moisture content of the microneedle patch can be reduced to less than 3% simply by continuing to dehydrate in the protector for 3-5 days. At this water content, the microneedle patch has the best structural strength and requires no extra time and cost.

In some embodiments, the dehydration operation is cold air dehydration, specifically, air drying at -40-35°C for 3-4 hours. Meanwhile, the wind speed is controlled within the range of 0.5-5 m/s to prevent the molding liquid from blowing out of the microneedle molding die and to prevent the curing rate from being lowered by an excessively low wind speed, both of which will have an impact on production efficiency.

In some embodiments, the protector includes a bubble cap and a patch plate, the bubble cap is arranged on the patch plate, and the microneedle patch is arranged in the bubble cap.

The bubble cap is attached to the patch to form a good protective structure, which can prevent the microneedle patch from being damaged by external force, and at the same time, it also has a good blocking effect, which can isolate external pollution sources such as bacteria and viruses and meet the aseptic requirements of GMP regulations.

The material of the bubble cap and/or patch is a barrier material with a water vapor permeability of 1-20 g/m²•24h.The water vapor permeability in this range can ensure the relatively fast dehydration rate of the microneedle patch, and meanwhile, it can also ensure that external bacteria are not easy to pollute through the protector. In some embodiments, the material of the bubble cap is polyvinyl chloride or polyethylene terephthalate. In some embodiments, the material of the bubble cap may also be polyester aluminized film, polyethylene, biaxially stretched polypropylene, aluminum foil, polyvinylidene chloride coated polypropylene and aluminized unstretched polypropylene film.

In some embodiments, the material of the patch plate is a spunbonded olefin or an aluminum-plastic film. In some embodiments, the materials of the patch plate may also be non-woven high-density polyethylene film, polyester aluminized film, polyethylene, biaxially stretched polypropylene, aluminum foil, polyvinylidene chloride coated polypropylene and aluminized unstretched polypropylene film.

In some embodiments, the prepared microneedle patch is a layered microneedle, and the preparation process of the microneedle patch includes:
the forming liquid includes a basement membrane liquid and a needle tip liquid; obtaining the above-mentioned microneedle formulation; taking a part of the microneedle formulation as basement membrane liquid, and adding a pharmaceutical active ingredient to another part of the microneedle formulation as a needle tip liquid;
applying the needle tip liquid into a microneedle cavity of a microneedle forming die, dehydrating and shrinking to obtain a microneedle tip part, proceed to applying the basement membrane liquid on the microneedle forming die, dehydrating to obtain a needle body part and a basement membrane connected with the needle body part, dehydrating until the water content is 3-15%, and demoulding to obtain a microneedle patch; and
placing the microneedle patch into a protector and put it in a sealed bag with a dehydrating agent to reduce the water content of the microneedle patch to 3%.

In some embodiments, the prepared microneedle patch is an integrated microneedle, and the preparation process of the microneedle patch includes:
obtaining the above-mentioned microneedle formulation; adding a pharmaceutical active ingredient into the microneedle formulation as a molding liquid;
applying the molding liquid on a microneedle molding die, dehydrating to obtain a microneedle and a basement membrane connected with the microneedle, dehydrating until the water content is 3-15%, and demoulding to obtain a microneedle patch; and
placing the microneedle patch into a protector and enclosing it in a sealed bag with a dehydrating agent to reduce the water content of the microneedle patch to 3%.

In some embodiments, the microneedle on the microneedle patch have a conical structure, such as a round sharp cone, an oval sharp cone, a regular polygonal sharp cone, an irregular polygonal sharp cone and the like. The height of the microneedle is 0.001 µm-1000 µm, and the maximum diameter is 0.005-3000 µm. Meanwhile, in order to ensure that the microneedle array has a certain density, the distance between the microneedles is 4 µm-1000 µm.

In some embodiments, the microneedle molding die includes a support plate and a plurality of microneedle molding areas. The shape of the microneedle molding area is consistent with that of the basement membrane of the microneedle patch to be prepared. And the microneedle molding areas are embedded in the support plate at intervals, the support plate is made of rigid materials, and the microneedle molding areas are made of flexible and breathable materials.

In some embodiments, the rigid material is selected from monocrystalline silicon, stainless steel, aluminum plate, titanium plate, silicate glass, quartz glass, ceramics, polytetrafluoroethylene, polyether-ether-ketone (PEEK), pyridinium propyl sulfobetaine, etc., and the flexible material is selected from siloxane.

The embodiment adopts the combination of flexible materials and rigid materials to prepare a microneedle forming die, and the microneedle molding area of flexible materials is fixed on a support plate of rigid materials, so that the support plate has a supporting and fixing function for the microneedle molding area and is used for maintaining the stability of the microneedle molding area. The microneedle cavity arranged on the microneedle molding area is used for curing and molding of microneedles, and the use of flexible materials is beneficial to reducing the stress during the microneedle demoulding process and improving the integrity of the microneedle after demoulding; meanwhile, the shape of the microneedle molding area is fixed by the support plate made of rigid materials, so that the microneedle molding area can be prevented from being deformed during the curing and shrinkage of the molding liquid, and the molding effect and demoulding integrity of the microneedle can be effectively improved.

In some embodiments, when the molding liquid is applied to the microneedle molding die, the microneedle molding die is vacuumized to promote the filling rate of the microneedle molding die and avoid leaving bubbles in the molded microneedle.

In a different embodiment, when the molding liquid is applied, the molding liquid can be introduced into the microneedle molding die by means of pressurized injection, atomized injection, roller coating, brush coating, injection, silk-screen printing and scraping coating.

In a preferred embodiment, the molding liquid is applied in the microneedle molding die by scraping coating.

The present application will be further illustrated with embodiments.

**Table 1**

| Items | First polyvin yl alcohol | Viscosity of the first polyvinyl alcohol mPas | Second polyvin yl alcohol | Viscosit y of the second polyvin yl alcohol mPas | Hydr oxyet hyl cellul ose | First polyv inylp yrroli done | Molecul ar weight of the first polyviny 1pyrrolid one | Seco nd poly vinyl pyrr olido ne | Molecul ar weight of the second polyviny lpyrrolid one | Sucr ose | Treh alose | Sodi um citrat e | Sodi um chlor ide | Sodiu m hydro xide |
|---|---|---|---|---|---|---|---|---|---|---|---|---|---|---|
| Embodiment 1 | 0.5 | 2.4 | 1.5 | 17.2 | 5.0 | 2.5 | 3500 | 7.0 | 210000 | 1.0 | 10.0 | 0.3 | 0.5 | 0.002 |
| Embodiment 2 | 0.8 | 2.8 | 1.8 | 22.4 | 5.4 | 2.8 | 10000 | 7.5 | 350000 | 1.3 | 10.5 | 0.4 | 0.7 | 0.003 |
| Embodiment 3 | 1.1 | 4.8 | 2.1 | 28.2 | 5.7 | 3.2 | 45000 | 8.0 | 600000 | 1.7 | 11.0 | 0.5 | 1.0 | 0.005 |
| Embodiment 4 | 1.4 | 6.2 | 2.4 | 31.7 | 6.2 | 3.7 | 60000 | 8.5 | 800000 | 2.0 | 11.5 | 0.5 | 1.2 | 0.006 |
| Embodiment 5 | 1.7 | 8.4 | 2.7 | 39.5 | 6.5 | 4.2 | 100000 | 9.0 | 1150000 | 2.3 | 12.0 | 0.6 | 1.5 | 0.007 |
| Embodiment 6 | 2.0 | 10.5 | 3.1 | 41.2 | 6.8 | 4.5 | 120000 | 9.5 | 1300000 | 2.6 | 12.5 | 0.7 | 1.7 | 0.009 |
| Embodiment 7 | 2.4 | 12.3 | 3.5 | 42.6 | 7 | 5 | 150000 | 10 | 1500000 | 3.0 | 13.0 | 0.8 | 2.0 | 0.010 |
| Comparative example 1 | 0 | - | 2 | 23.5 | 5.0 | 2.5 | 3500 | 7.0 | 500000 | 1.0 | 10.0 | 0.3 | 0.5 | 0.002 |
| Comparative example 2 | 2 | 2.4 | 0 | - | 5.0 | 2.5 | 3500 | 7.0 | 500000 | 1.0 | 10.0 | 0.3 | 0.5 | 0.002 |
| Comparative example 3 | 0.5 | 2.4 | 1.5 | 23.5 | 5.0 | 0 | - | 9.5 | 500000 | 1.0 | 10.0 | 0.3 | 0.5 | 0.002 |
| Comparative example 4 | 0.5 | 2.4 | 1.5 | 23.5 | 5.0 | 9.5 | 3500 | 0 | - | 1.0 | 10.0 | 0.3 | 0.5 | 0.002 |

**Table 2**

| Items | Water addition of Group A | Water addition of Group B | Water content of the first dehydration % | Water content of following dehydration % | Duration of first dehydration min |
|---|---|---|---|---|---|
| Embodiment 1 | 15 | 12 | 3 | 1 | 192 |
| Embodiment 2 | 25 | 20 | 5 | 1 | 221 |
| Embodiment 3 | 35 | 28 | 7 | 1 | 203 |
| Embodiment 4 | 45 | 36 | 9 | 2 | 205 |
| Embodiment 5 | 55 | 44 | 11 | 2 | 211 |
| Embodiment 6 | 65 | 52 | 13 | 2 | 218 |
| Embodiment 7 | 75 | 60 | 15 | 2 | 224 |
| Comparative example 1 | 75 | 60 | 15 | 3 | 217 |
| Comparative example 2 | 75 | 60 | 15 | 3 | 202 |
| Comparative example 3 | 75 | 60 | 15 | 3 | 209 |
| Comparative example 4 | 75 | 60 | 15 | 3 | 196 |
| Comparative example 5 | 75 | 60 | 15 | - | 473 |

### Embodiment 1

The embodiment is used for explaining the microneedle patch disclosed by the appliation and the preparation method thereof. The method includes the following steps:
weighing 0.5 parts of the first polyvinyl alcohol (viscosity: 2.4 mPas), 1.5 parts of the second polyvinyl alcohol (viscosity: 17.2 mPas), 5 parts of hydroxyethyl cellulose, 2.5 parts of the first polyvinylpyrrolidone (molecular weight: 3500) and 7 parts of the second polyvinylpyrrolidone (molecular weight: 210000) by weight, adding them into 15 parts of water, then heating and dispersing at 90°C to obtain a component A, and cooling.
weighing 1 part of sucrose, 10 parts of trehalose, 0.3 parts of sodium citrate, 0.5 parts of sodium chloride and 0.002 parts of sodium hydroxide by weight, dissolving them in 12 parts of water to obtain a component B; and
mixing the component A and component B to obtain a microneedle formulation.

Obtaining the above-described microneedle formulation; Taking a part of the microneedle formulation as a basement membrane liquid, and adding a pharmaceutical active ingredient to another part of the microneedle formulation as a needle tip liquid;
scraping the needle tip liquid in a microneedle cavity of a microneedle molding die, dehydrating and shrinking to obtain a needle tip part of the microneedle, continuously scraping the basement membrane liquid on the microneedle molding die, dehydrating to obtain a needle body part and a basement membrane connected with the needle body part, preliminarily dehydrating until the water content is 3%, and demoulding to obtain a microneedle patch;
placing the microneedle patch in a protector, and enclosing in an aluminum-plastic film with a dehydrating agent to continue dehydration. After letting stand for 3 days, the water content of the resulting microneedle patch dropped to 1%.

### Embodiments 2-7

Embodiments 2-7 are used to illustrate the microneedle patch and its preparation method disclosed in the present application, including most steps from Embodiment 1, with the following differences:
the substance content components for Embodiments 2-7 were adopted according to Table 1, and the water content of the first dehydration and the water content of the following dehydration of Embodiments 2-7 are shown in Table 2.

### Comparative examples 1-4

Comparative examples 1- 4 are used to explain the microneedle patch and its preparation method disclosed in the present application, including most steps from Embodiment 1, with the following differences:
the substance content components for Comparative examples 1-4 were adopted according to Table 1, and the first dehydration water content and the water content of the following dehydration of Comparative examples 1-4 are shown in Table 2.

### Comparative example 5

Comparative example 5 are used to explain the microneedle patch and its preparation method disclosed in the present application, including most steps from Embodiment 1, with the following differences:
only the preliminary dehydration was adopted for Comparative example 5, and the water-permeable protector and dehydrating agent were not used for the following drying steps, and reduced the water content was lowered to 1% directly, but it took unequal time.

### Performance test

1. The universal material testing machine directly presses the needle to test the strength: the microneedle patches prepared from the above-mentioned Embodiments 2-7 and Comparative examples 1-4 were placed on the universal material experimental platen, the machine was started, and the pressure sensor moved downward, the breaking point pressure was recorded when the pressure sensor detected that the microneedle broke. The diameter of the pressure head on the pressure sensor was 8mm, and the total number of the needles pressed was about 194. The experimental results are as follows.

| Embodiment/Comparative example | Breaking point pressure (N) | Breaking pressure of single needle (N) |
|---|---|---|
| Embodiment 1 | 136.43 | 0.70 |
| Embodiment 2 | 122.30 | 0.63 |
| Embodiment 3 | 134.77 | 0.69 |
| Embodiment 4 | 136.45 | 0.73 |
| Embodiment 5 | 131.17 | 0.68 |
| Embodiment 6 | 132.65 | 0.71 |
| Embodiment 7 | 135.77 | 0.66 |
| Comparative example 1 | 108.62 | 0.42 |
| Comparative example 2 | 102.45 | 0.39 |
| Comparative example 3 | 110.71 | 0.53 |
| Comparative example 4 | 117.19 | 0.46 |

It can be seen that the microneedle patch prepared by the method provided by the present application obviously has higher microneedle structural strength. Comparative examples 1-4 show that the lack of any one of the first polyvinyl alcohol, the second polyvinyl alcohol, the first polyvinylpyrrolidone and the second polyvinylpyrrolidone will lead to the deterioration of the strength of microneedles. This indicates that the first polyvinyl alcohol, the second polyvinyl alcohol, the first polyvinylpyrrolidone and the second polyvinylpyrrolidone have played a good synergistic role in improving the strength of microneedles.

2. Sealing film puncture experiment for parafilm: the microneedle patches from Embodiment 1 and Comparative examples 1-4 were selected, the multiple layers of parafilm were stacked, the microneedle patch with the needle tip was arranged to face the parafilm and placed under the universal material testing machine, and the microneedle was pressed with a force of 147N (approximately the pressing force of an adult with fingers) to make the microneedle penetrate into the parafilm. After the experiment, the parafilm was taken out, and the parafilm was soaked with red ink. By obeserving whether the red ink could penetrate the parafilm, the number of layers of the parafilm was determined.

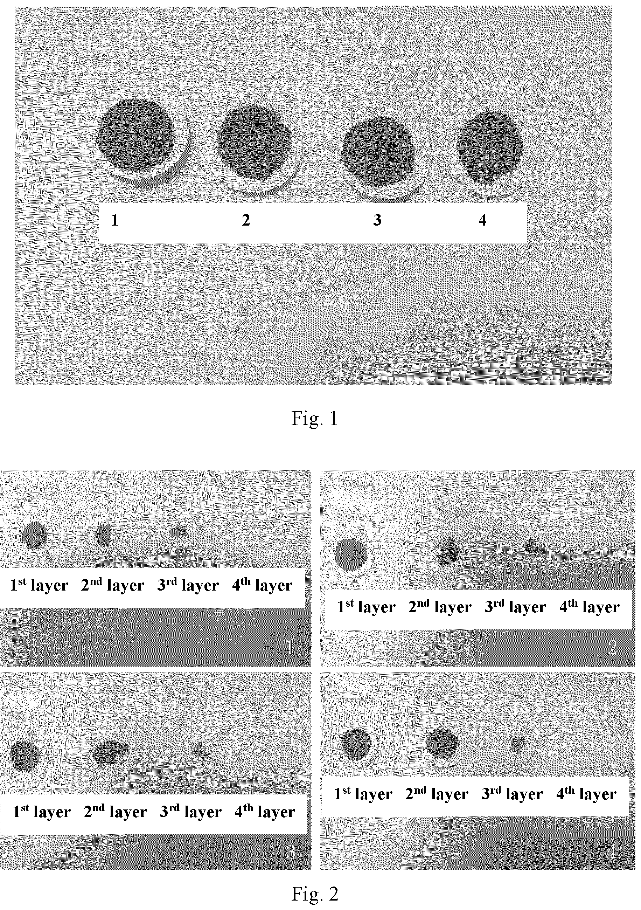

The results obtained from Embodiment 1 are shown in Fig. 1, and those obtained from Comparative examples 1-4 are shown in Fig. 2.

As can be seen from Fig. 1 and Fig. 2, after the microneedle patch of embodiment 1 is punctured with the parafilm sealing film, the red ink can be impregnated to the fourth layer. It can be determined that the microneedle has penetrated the fourth layer of parafilm at 147N.

The ones from Comparative examples 1-4 can only penetrate the third layer, and there is no infiltration on the fourth layer.

3. Leaf puncture: the microneedle patches of Embodiment 1 and Comparative examples 1-4 were selected, the microneedle patches were applied to penetrate the leaves (Zamioculcas zamiifolia), the microneedle patches were removed after completion, and the microneedle puncture on the surface of the leaves was observed under a microscope.

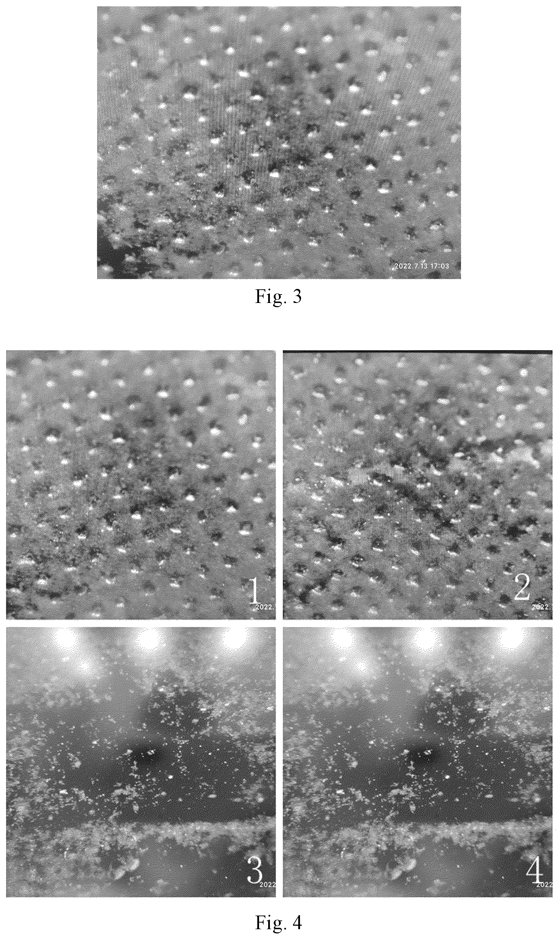

The leaf puncture results of the microneedle patches of Embodiment 1 are shown in Fig. 3, and those of Comparative examples 1-4 are shown in Fig. 4. It can be seen that the microneedle patches provided by Embodiment 1 can completely penetrate the leaves with a deep penetration depth, while Comparative examples 1-2 have a shallow penetration depth, and the ones from Comparative examples 3-4 are shallower.

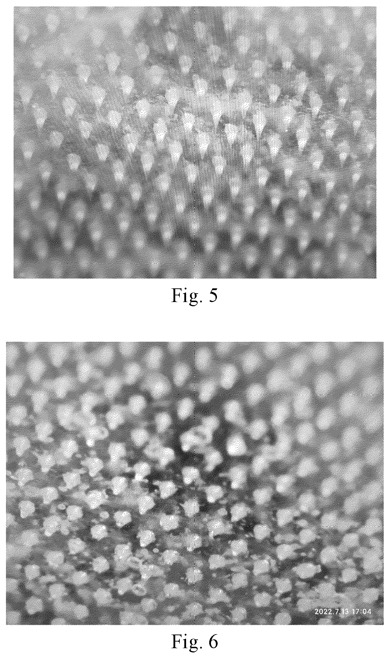

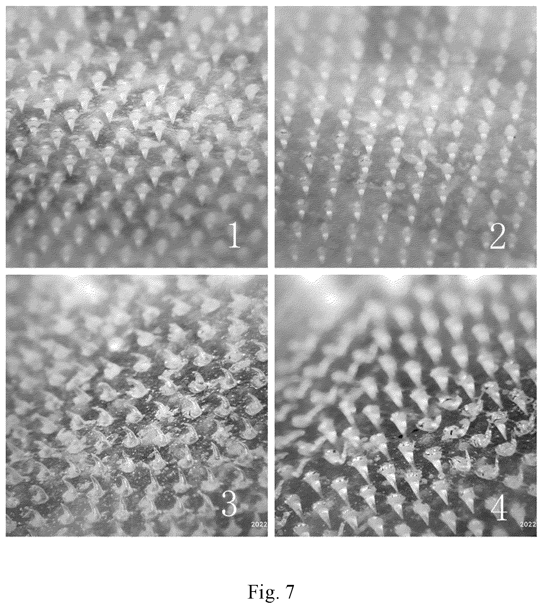

The morphology of the microneedle patch of Embodiment 1 before penetration is shown in Fig. 5, and the morphology after penetration is shown in Fig. 6. The morphology of the microneedle patches of Comparative examples 1-4 before penetration is shown in Fig. 7, and the morphology after penetration is shown in Fig. 8.

As can be observed, the microneedle patch of Embodiment 1, some complete needle forms can still be seen after penetration, while the microneedle patches of Comparative Examples 1-4 basically do not display any complete needle forms after penetration.

The above are merely the preferred embodiments of this application, and are not intended to limit the application. Any modification, equivalent substitution and improvement made within the spirit and principle of this application shall be included in the protection scope of this application.

## Claims

1. A microneedle formulation, comprising a component A as follows:
polyvinyl alcohol, polyvinylpyrrolidone and hydroxyethyl cellulose;
wherein the polyvinyl alcohol comprises a first polyvinyl alcohol and a second polyvinyl alcohol, and the first polyvinyl alcohol has a viscosity of 2.4-12.3 mPas, and the second polyvinyl alcohol has a viscosity of 17.2-42.6 mPas; and
wherein the polyvinylpyrrolidone comprises a first polyvinylpyrrolidone and a second polyvinylpyrrolidone, the first polyvinylpyrrolidone has a molecular weight of 3500-150000, and the second polyvinylpyrrolidone has a molecular weight of 210000-1500000.

2. The microneedle formulation of claim 1, wherein the first polyvinylpyrrolidone has a viscosity of 0.8-10.6 mPas, and the second polyvinylpyrrolidone has a viscosity of 16.5-88.4 mPas.

3. The microneedle formulation of claim 1, wherein the component A comprises the following components by weight:
2-6 parts of polyvinyl alcohol, 9.5-15 parts of polyvinylpyrrolidone and 5-7 parts of hydroxyethyl cellulose;
wherein, 0.5-2.5 parts of first polyvinyl alcohol and 1.5-3.5 parts of second polyvinyl alcohol; and
2.5-5 parts of first polyvinylpyrrolidone and 7-10 parts of second polyvinylpyrrolidone.

4. The microneedle formulation of claim 1, wherein the component A further comprises 35-49 parts by weight of a first solvent.

5. The microneedle formulation of claim 1, wherein the microneedle formulation further comprises a component B as follows:
sugar, buffer and salt.

6. The microneedle formulation of claim 5, wherein the component B comprises the following components by weight:
11-16 parts of sugar, 0.3-0.8 parts of buffer and 0.5-2 parts of salt.

7. The microneedle formulation of claim 6, wherein the sugar comprises 1-3 parts by weight of sucrose and 10-13 parts by weight of trehalose, and the component B further comprises 0.002-0.01 part by weight of sodium hydroxide and 14-17 parts by weight of a second solvent.

8. The microneedle formulation of claim 5, wherein a weight ratio of the component A to the component B is (16.5-77): (11.8-35.81).

9. The microneedle formulation of claim 1, wherein the microneedle formulation further comprises a pharmaceutical active ingredient, and a weight ratio of the component A to the pharmaceutical active ingredient is (16.5-77): (0.6-22.46).

10. A preparation method for a microneedle patch, comprising:
obtaining a pharmaceutical active ingredient and the microneedle formulation of any one of claims 1 to 8, and preparing a molding liquid for microneedle molding;
applying the molding liquid on a microneedle molding die, dehydrating at -40-35°C until a water content is 3-15%, and demoulding to obtain a microneedle patch;
placing the microneedle patch in a protector and enclosing it in a sealed bag with a dehydrating agent to lower the water content of the microneedle patch to 3%.

11. The preparation method of claim 10, wherein the protector comprises a bubble cap and a patch plate, wherein the bubble cap is arranged on the patch plate, and the microneedle patch is arranged in the bubble cap.

12. The preparation method of claim 10, wherein the sealed bag has a water vapor permeability of less than 0.8 g/m²•24h.

13. The preparation method of claim 12, wherein the sealed bag is a mixed film of one or more of biaxially oriented polypropylene, polyethylene terephthalate, chlorinated polypropylene resin and aluminum foil.

14. The preparation method of claim 11, wherein the material of the bubble cap and/or the patch plate is a barrier material with a water vapor permeability of 1-20 g/m²•24h.

15. The preparation method of claim 14, wherein the material of the bubble cap is polyvinyl chloride or polyethylene terephthalate.

16. The preparation method of claim 12, wherein the material of the patch plate is a spunbonded olefin or an aluminum-plastic film.
